Europäisches Patentamt

(19) European Patent Office

Office européen des brevets

(11) Numéro de publication : **0 335 426 B1**

# (12) FASCICULE DE BREVET EUROPEEN

(45) Date de publication du fascicule du brevet :
03.07.91 Bulletin 91/27

(51) Int. Cl.⁵ : **A61K 37/02, A61K 39/395, A61K 39/44, G01N 33/569**

(21) Numéro de dépôt : 89108697.7

(22) Date de dépôt : 13.09.85

(54) **Composition et procédé pour protéger les lymphocytes T contre l'agent étiologique des lymphadénopathies et du syndrome d'immunisation acquise.**

(30) Priorité : 14.09.84 FR 8414172

(43) Date de publication de la demande :
04.10.89 Bulletin 89/40

(61) Numéro de publication de la demande initiale en application de l'article 76 CBE : 0176429

(45) Mention de la délivrance du brevet :
03.07.91 Bulletin 91/27

(84) Etats contractants désignés :
AT BE CH DE GB IT LI LU NL SE

(56) Documents cités :
EP-A- 0 018 794
NATURE, vol. 312, 27 décembre 1984, pages 767-768; D. KLATZMANN et al.: "T-lymphocyte T4 molecule behaves as the receptor for human retrovirus LAV"
Proceedings of the IV International Conference on AIDS, Stockholm 12-16 June 1988, Abs.1524,3062,3082,3088,3518,3618,3521,3522,3520,Science vol.242 (25.11.88) pp.1166-68.

(73) Titulaire : INSTITUT PASTEUR
25-28, rue du Docteur Roux
F-75724 Paris Cédex 15 (FR)
Titulaire : CENTRE NATIONAL DE LA RECHERCHE SCIENTIFIQUE (CNRS)
15, Quai Anatole France
F-75007 Paris (FR)
Titulaire : UNIVERSITE PIERRE ET MARIE CURIE PARIS VI
4, place Jussieu
F-75230 Paris Cedex 05 (FR)

(72) Inventeur : Klatzmann, David
84, Quai de Jemmapes
F-75010 Paris (FR)
Inventeur : Gluckman, Jean-Claude
70 Bld. du Port Royal
F-75005 Paris (FR)
Inventeur : Montagnier, Luc
21, rue de Malabry
F-92350 Le Plessis Robinson (FR)

(74) Mandataire : Gutmann, Ernest et al
S.C. Ernest Gutmann - Yves Plasseraud 67, boulevard Haussmann
F-75008 Paris (FR)

**Description**

L'invention est relative à un procédé et à des compositions pour protéger les lymphocytes T contre l'agent étiologique des lymphadénopathies et du syndrome d'immunodépression acquise.

L'agent étiologique des affections susdites a récemment été identifié. Il s'agit d'un virus qui a été dénommé LAV. Ce virus et divers variants de ce virus ont été complètement identifiés dans la demande de brevet européen déposée le 14 septembre 1984 au nom de l'INSTITUT PASTEUR et sous le titre "Antigènes, moyens et méthode pour le diagnostic de lymphadénopathie et du syndrome d'immunodépression acquise", sous la priorité de la demande de brevet britannique n° 83.24800 déposée le 15 septembre 1983 (EP-A-0138667). On sait par ailleurs qu'un virus du même type a été isolé aux Etats-Unis d'Amérique. Il a été dénommé HTVL-III (SCIENCE, 1984, Vol. 224, 4648, 497). Pour la commodité du langage, il sera fait référence dans ce qui suit au virus LAV, étant entendu que cette désignation s'étend en fait à tous les virus doués de propriétés équivalentes.

Il avait déjà été reconnu que les lymphocytes T-auxiliaires, plus particulièrement les cellules porteuses d'un déterminant OKT4+, ou "lymphocytes T4+", selon la désignation qui sera utilisée ci-après (cellules 3 Leu), semblaient former la principale cible du virus. Ces lymphocytes ont la particularité d'être reconnaissables par différents anticorps monoclonaux commercialisés par la firme ORTHO PHARMACEUTICAL CORPORATION sous la désignation OKT4. Ces anticorps monoclonaux ont été décrits plus particulièrement dans la demande de brevet européen n° 0018794 déposée le 25 avril 1980. Il a également été établi que cet anticorps monoclonal reconnaissait plus particulièrement une protéine de poids moléculaire de l'ordre de 62.000 daltons, révélée après immunoprécipitation à partir d'un lysat de thymocytes humains marqués au borohydrure de sodium tritié, et ce après une oxydation douce en présence d'une faible concentration de périodate de sodium (C. TERHORST et coll., SCIENCE (1980), 209, 520-521). Cette protéine a été dénommée "antigène T4" par les auteurs susdits. Elle sera ci-après encore désignée comme "protéine T4". En particulier cette protéine T4 est reconnue par un anticorps monoclonal secrété par l'hybridome qui a été déposé par la société ORTHO PHARMACEUTICAL CORPORATION auprès de la collection dite "American Type Culture Collection" 12301 Parklawn Drive, ROCKVILLE, MD 20852, aux Etats-Unis le 28 avril 1979. Cet hybridome est catalogué sous le n° ATCC CRL 8002.

L'invention découle de la découverte que cette protéine T4 joue apparemment un rôle essentiel au niveau du processus d'infection des lymphocytes T par les susdits virus (ci-après plus généralement dénommés LAV). Plus particulièrement le processus d'infection des lymphocytes T paraît tributaire de l'interaction du virus avec la molécule T4, celle-ci paraissant jouer un rôle de récepteur pour le virus à la surface de la membrane. La reconnaissance de cette interaction découle de la constatation qui a été faite que l'infection d'une culture de cellules de lymphocytes T4 par les virus LAV dans des conditions expérimentales qui normalement l'autorisent, ne se produit plus en présence d'anticorps monoclonaux anti-T4.

Il n'est en particulier plus possible de détecter l'immunofluorescence caractéristique de l'infection dans les conditions de culture décrites par E. L. REINHERZ et coll., Proc. Natl. Acad. Sci. USA (1979), Vol. 76, 4061-4065, dès que le milieu de culture contient également des anticorps monoclonaux anti-T4. Au contraire, des anticorps monoclonaux dirigés contre des antigènes autres que la protéine T4 présente sur les lymphocytes T ne perturbent en aucune façon l'infection dans les mêmes conditions des lymphocytes T par les virus LAV.

D'une façon générale l'invention concerne des compositions dont le but est d'interférer avec le processus infectieux, que ce soit au niveau de la pénétration des virus LAV dans les lymphocytes T ou de l'inhibition ou de la destruction du virus infectieux à l'intérieur même de lymphocytes T infectés.

L'élément caractéristique de la composition est constitué par la protéine T4 ou d'un polypeptide comprenant le site de fixation de la protéine T4 au virus LAV, en association avec tout véhicule destiné à faciliter la mise en contact des lymphocytes T *in vitro* ou *in vivo* avec ladite composition.

La protéine T4 peut être isolée à partir de cultures de lymphocytes T4, par exemple dans les conditions décrites par C. TERHORST et coll. ou E.L. REINHERZ et coll. (documents déjà cités). L'invention concerne encore des compositions médicamenteuses contenant des fragments peptidiques obtenus à partir de cette protéine T4, dans la mesure où ceux-ci comportent le site de fixation du virus. Ces protéines ou polypeptides, qui peuvent alors entrer en compétition avec les lymphocytes T4 dans la fixation du virus, peuvent contribuer à prévenir l'infection virale ou même à l'interrompre, lorsque celle-ci se trouve déjà à un état avancé. Il va de soi que, de préférence, les protéines T4 mises en oeuvre (ou les polypeptides qui portent le même site de fixation du virus) seront de préférence conformes à ceux qui appartiennent aux lymphocytes humains. On remarquera que des polypeptides portant ce site de fixation peuvent être obtenus par fragmentation de la protéine T4, notamment avec des enzymes susceptibles de reconnaître des liaisons particulières (par exemple l'achromase, la trypsine, l'alpha-chymotrysine, etc...) et sélection des fragments obtenus par interaction avec des anticorps monoclonaux anti-T4.

Dans l'un de ses premiers modes de réalisation, l'invention concerne donc une composition pharmaceutique, de préférence injectable, constituée par l'association d'une protéine telle qu'elle a été définie ci-dessus, ou par un polypeptide, notamment fragment de protéine T4, doué de propriétés équivalentes, en association avec un excipient pharmaceutiquement acceptable (notamment d'une solution aqueuse, injectable, stérile, de préférence isotonique). Il va de soi que la composition doit être dosée de façon à assurer son efficacité au niveau de la prévention ou de l'inhibition *in vivo* de l'infection possible des lymphocytes T4 par le virus LAV. Plus particulièrement on observera que l'inhibition de l'infection supposera la réalisation d'une compétition efficace en faveur de la substance active vis-à-vis des virus, à l'égard desdits récepteurs.

La composition peut être utilisée soit seule, notamment lorsqu'elle est utilisée à titre préventif, soit en association ou en combinaison avec d'autres agents.

L'invention peut également être mise en oeuvre in vitro, par exemple sur des prélèvements de lymphocytes contenant des cellules infectées obtenues à partir du patient, ces lymphocytes infectés étant alors mis en contact avec les compositions selon l'invention pendant le temps nécessaire à la destruction du virus ou des cellules infectées, avant d'être regreffés au patient.

L'invention concerne encore des protéines T4 ou polypeptides du type sus-indiqué, à l'état fixé sur une résine appropriée à la réalisation de chromatographies affines, autrement dit d'un conjugué entre ces protéines T4 ou polypeptides et ladite résine, pour l'élimination du virus LAV contenu dans le plasma d'un patient affecté des susdites maladies, ce procédé étant caractérisé en ce que l'on fait passer ce plasma sur une colonne d'affinité comportant ce conjugué et en ce que l'on recueille le plasma ainsi débarrassé de sa teneur en virus LAV. La colonne porteuse de molécules T4 peut servir également à piéger les anticorps anti-T4 résultant d'un mécanisme auto-immunitaire quelquefois impliqué dans la maladie.

Les anticorps anti-T4 ainsi piégés sont en général constitués par des auto-anticorps présents dans le plasma. Cette opération peut être réalisée par incorporation de la colonne d'affinité dans un circuit extra-corporel, le sang à purifier étant prélevé sur le malade puis, après purification, réinjecté dans le malade.

L'invention concerne enfin les différentes compositions évoquées ci-dessus, en tant que réactifs pour l'étude in vitro, pour l'étude du comportement du virus LAV par rapport à des lymphocytes T4 dans des conditions déterminées.

**Revendications**

1. Composition pharmaceutique caractérisée par l'association d'une molécule T4 consistant, soit en la protéine T4, soit en un polypeptide contenant le site de fixation de la protéine T4 pour un virus LAV, cette molécule T4 étant associée au sein de ladite composition avec un véhicule pharmaceutiquement acceptable.

2. Composition selon la revendication 1, caractérisée en ce que la molécule T4 consiste en un fragment de la protéine T4, apte à entrer en compétition avec les lymphocytes T4 dans la fixation du virus LAV.

3. Composition selon la revendication 1, caractérisée en ce que la molécule T4 consiste en un fragment de la protéine T4 présentant la capacité d'être reconnue par des anticorps monoclonaux anti-T4, notamment du type de ceux décrits dans la demande de brevet européen n° 0018794 et qui sont sécrétés par l'hybridome ATCC CRL 8002.

4. Composition pharmaceutique selon l'une quelconque des revendications 1 à 3, caractérisée en ce qu'elle est applicable à la prévention ou à l'interruption d'une infection virale due à un virus LAV.

5. Conjugué entre, d'une part, la molécule T4 consistant, soit en la protéine T4, soit en un polypeptide contenant le site de fixation de la protéine T4 pour un virus LAV et, d'autre part, une résine appropriée à la réalisation de chromatographies affines pour la mise en oeuvre d'un procédé d'élimination du virus LAV, et le cas échéant d'anticorps anti-T4 résultant d'un mécanisme auto-immunitaire, contenus dans le plasma d'un patient atteint de lymphadénopathie ou du syndrome d'immunodépression acquise, ce procédé comprenant les étapes consistant à faire passer ce plasma sur une colonne d'affinité contenant le susdit conjugué et à recueillir le plasma ainsi débarrassé de sa teneur en virus LAV et, le cas échéant, en anticorps anti-T4.

6. Conjugué selon la revendication 5, caractérisée en ce que la molécule T4 consiste en un fragment de la protéine T4 apte à entrer en compétition avec les lymphocytes T4 dans la fixation du virus LAV.

7. Conjugué selon la revendication 5, caractérisée en ce que la molécule T4 consiste en un fragment de la protéine T4 présentant la capacité d'être reconnue par des anticorps monoclonaux anti-T4, notamment du type de ceux décrits dans la demande de brevet européen n° 0018794 et qui sont sécrétés par l'hybridome ATCC CRL 8002.

8. Procédé pour la préparation d'une composition permettant de prévenir ou d'interrompre in vivo l'infection des lymphocytes T avec un virus LAV, caractérisé en ce que ladite composition contient, en association avec un véhicule pharmaceutiquement acceptable, une molécule T4 consistant soit en la protéine T4, soit en un polypeptide contenant le site de fixation de la protéine T4 pour un virus LAV, cette molécule T4 étant associée au sein de ladite composition avec un véhicule pharmaceutiquement acceptable.

9. Procédé selon la revendication 8, caractérisé en ce que la molécule T4 consiste en un fragment de la protéine T4, apte à entrer en compétition avec les lymphocytes T4 dans la fixation du virus LAV.

10. Procédé selon la revendication 8, caractérisé en ce que la molécule T4 consiste en un fragment de la protéine T4 présentant la capacité d'être reconnue par des anticorps monoclonaux anti-T4, notamment du type de ceux décrits dans la demande de brevet européen n° 0018794 et qui sont sécrétés par l'hybridome ATCC CRL 8002.

## Ansprüche

1. Pharmazeutische Zusammensetzung gekennzeichnet durch die Assoziation eines Moleküls T4 bestehend entweder aus dem Protein T4 oder aus einem Polypeptid, das die Fixierungsstelle des Proteins T4 für ein LAV-Virus enthält, wobei dieses Molekül T4 in der genannten Zusammensetzung mit einem pharwazeutisch verträglichen Transportmittel zusammen gesetzt ist.

2. Zusammensetzung gemäß Anspruch 1, dadurch gekennzeichnet, daß das Molekül T4 aus einem Fragment des Proteins T4 besteht, das geeignet ist, bei der Fixierung des LAV-Virus zu den lymphocyten T4 in Konkurrenz zu treten.

3. Zusammensetzung gemäß Anspruch 1, dadurch gekennzeichnet, daß das Molekül T4 aus einem Fragment des Proteins T4 besteht, das die Fähigkeit aufweist, durch monoklonale Anti-T4-Antikörper erkannt zu werden, insbesondere vom Typ jener, die in der europäischen Patentanmeldung Nr. 0018794 beschrieben sind und die durch das Hybridom ATCC CRL 8002 ausgeschieden werden.

4. Pharmazeutische Zusammensetzung gemäß einem der Ansprüche 1 bis 3, dadurch gekennzeichnet, daß sie zur Vorbeugung oder zur Unterbrechung einer Virusinfektion durch ein LAV-Virus anwendbar ist.

5. Verbindung zwischen, einerseits, dem Molekül T4 bestehend entweder aus dem Protein T4 oder aus einem Polypeptid, das die Fixierungsstelle des Proteins T4 für ein LAV-Virus enthält und, andererseits, einem Harz, das zur Durchführung von Affinitätschromatographien geeignet ist, für die Anwendung eines Verfahrens zur Eliminierung des LAV-Virus und gegebenenfalls der Anti-T4-Antikörper, die von einem Autoimmunmechanismus resultieren, die im Plasma eines Patienten enthalten sind, der von lymphadenopathie oder vom erworbenen Immundepressionssyndrom befallen ist, wobei dieses Verfahren die Schritte umfaßt, die darin bestehen, das Plasma eine Affinitätskolonne passieren zu lassen, die die oben genannte Verbindung enthält und das dann von seinem Genalt an LAV-Virus und gegebenenfalls an Anti-T4-Antikörpern befreite Plasma wieder aufzunehmen.

6. Verbindung gemäß Anspruch 5, dadurch gekennzeichnet, daß das Molekül T4 aus einem Fragment des Proteins T4 besteht, das geeignet ist, bei der Fixierung des LAV-Virus mit den lymphocyten T4 in Konkurrenz zu treten.

7. Verbindung gemäß Anspruch 5, dadurch gekennzeichnet, daß das Molekül T4 aus einem Fragment des Proteins T4 besteht, das die Fähigkeit aufweist, von monoklonalen Anti-T4-Antikörpern erkannt zu werden, insbesondere vom Typ jener, die in der europäischen Patentanmeldung Nr. 0018794 beschrieben sind und die vom Hybridom ATCC CRL 8002 ausgeschieden werden.

8. Verfahren zur Herstellung einer Zusammensetzung, die es erlaubt, in vivo der Infektion der Lymphocyten T mit einem LAV-Virus vorzubeugen oder sie zu unterbrechen, dadurch gekennzeichnet, daß die genannte Zusammensetzung, in Verbindung mit einem pharmazeutisch verträglichen Transportmittel, ein Molekül T4 enthält, bestehend entweder aus dem Protein T4 oder einem Polypeptid, das die Fixierungstelle des Proteins T4 für ein LAV-Virus enthält, wobei dieses Molekül T4 in der genannten Zusammensetzung mit einem pharmazeutisch verträglichen Transportmittel zusammen gesetzt ist.

9. Verfahren gemäß Anspruch 8, dadurch gekennzeichnet, daß das Molekül T4 aus einem Fragment des Proteins T4 besteht, das geeignet ist, bei der Fixierung des LAV-Virus mit den Lymphocyten T4 in Konkurrenz zu treten.

10. Verfahren gemäß Anspruch 8, dadurch gekennzeichnet, daß das Molekül T4 aus einem Fragment des Proteins T4 besteht, das die Fähigkeit aufweist, durch monoklonale Anti-T4-Antikörper erkannt zu werden, insbesondere vom Typ jener, die in der europäischen Patenmanmeldung Nr. 0018794 beschrienen sind und die durch das Hybridom ATCC CRL 8002 ausgeschieden werden.

## Claims

1. Pharmaceutical composition characterized by the association of a T4 molecule consisting either of the T4 protein or of a polypeptide which contains the fixation site of the T4 protein on a LAV virus, said T4 molecule being associated in said composition with a pharmaceutically acceptable vehicle.

2. Composition according to claim 1, characterized in that the T4 molecule consists of a protein T4 fragment which is able to enter competition with T4 lymphocytes in respect of the fixation of the LAV virus.

3. Composition according to claim 1, characterized in that the T4 molecule consists of a fragment of the T4 protein which presents the ability of being recognized by monoclonal anti-T4 antibodies, particularly of the type of those disclosed in European patent application N° 0018794 and which are secreted by the hybridoma ATCC CRL 8002.

4. Pharmaceutical composition according to any one of claims 1 to 3, characterized in that it is applicable to the preventing or interrupting a viral infection due to LAV virus.

5. Conjugate between, on the one hand, the T4 molecule which consists either of the T4 protein or of a polypeptide which contains the fixation site of the T4 protein on a LAV virus, and on the other hand, a resin appropriate for the performance of affinity chromatographies for use in a process for removing the LAV virus, and, possibly, anti-T4 antibodies which result from an auto-immune mechanism, contained in the plasma of a patient affected with lymphadenopathy or with an acquired immunodepression syndrom, said process comprising the steps which consist in having said plasma passed onto an affinity column comprising said conjugate and in recovering the plasma so freed from its content of LAV virus and, possibly, of anti-T4 antibodies.

6. Conjugate according to claim 5, characterized in that the T4 molecule consits of a fragment of the T4 protein which is able to enter competition with T4 lympochytes in the fixation of the LAV virus.

7. Conjugate according to claim 5, characterized in that the T4 molecule consists of a fragment of the T4 protein which presents the capacity of being recognized by anti-T4 monoclonal antibodies, particularly of the type of those disclosed in European patent application N° 0018794 and which are secreted by the hybridoma ATCC CRL 8002.

8. Process for the preparation of a composition which enables an infection of T-lymphocytes with a virus LAV to be prevented or interrupted in vivo, characterized in that said composition contains, in association with a pharmaceutically acceptable vehicle, a T4 molecule consisting either of the T4 protein, or in a polypeptide containing the fixation site of the T4 protein in respect of a LAV virus, said T4 molecule being associated within said composition with a pharmaceutically acceptable vehicle.

9. Process according to claim 8, characterized in that the T4 molecule consists of a fragment of the T4 protein, able to enter competition with the T4 lymphocytes in respect of the fixation of the LAV virus.

10. Process according to claim 8, characterized in that the T4 molecule consists of a fragment of the T4 protein which presents the ability of being recognized by anti-T4 monoclonal antibodies, particularly of the type of those disclosed in European patent application N° 0018794 and which are secreted by the hybridoma ATCC CRL 8002.